# EUROPÄISCHE PATENTSCHRIFT

(11) **EP 2 574 343 B1**
(45) Veröffentlichungstag und Bekanntmachung des Hinweises auf die Patenterteilung: **29.01.2014**
(21) Anmeldenummer: 11007992.8
(22) Anmeldetag: 30.09.2011
(51) Int. Cl.: A61K 36/185, A61K 36/736, A61K 36/889, A61K 36/53, A61K 9/00, A61P 17/00, A61P 17/02, A61P 17/06, A61P 17/08, A61P 17/12, A61K 47/06, A61K 47/44

(54) **Zusammensetzung zum Auftragen auf die Haut und Verwendung derselben**
Compound for skin application and use of same
Composition destinée à l'application sur la peau et utilisation de celle-ci

(43) Veröffentlichungstag der Anmeldung: 03.04.2013
(73) Patentinhaber: Alnapharm GmbH & Co.KG, 22399 Hamburg (DE)
(72) Erfinder: Nahavandi, Ali, 22399 Hamburg (DE)
(74) Vertreter: Scholz, Volker

(56) Entgegenhaltungen:
- DE-A1-102007 036 499
- US-A- 5 997 889
- US-A1- 2002 012 642
- US-A1- 2005 100 524
- US-A1- 2005 226 945
- US-A1- 2008 113 033
- DATABASE WPI Week 200025 Thomson Scientific, London, GB; AN 2000-291326 XP002669271, & RU 2 127 584 C1 (KONOVALOV V N) 20. März 1999 (1999-03-20)
- SRIVASTAVA P ET AL: "Burn wound healing property of Cocos nucifera: An appraisal", INDIAN JOURNAL OF PHARMACOLOGY 20081001 IN, Bd. 40, Nr. 4, 1. Oktober 2008 (2008-10-01), Seiten 144-146, XP009156408, ISSN: 0253-7613

## Beschreibung

Die vorliegende Erfindung betrifft eine Zusammensetzung zum Auftragen auf die Haut sowie deren Verwendung bei der Behandlung von Hauterkrankungen.

Schuppenflechte (Psoriasis) und Neurodermitis (atopisches Ekzem) sind sehr häufig vorkommende, chronische, nicht-ansteckende, entzündliche Hautkrankheiten.

So leiden beispielsweise etwa 2 bis 3% der Bevölkerung an der am häufigsten vorkommenden Psoriasis vulgaris, während Psoriasis pustolosa ebenfalls bekannt ist. Frauen und Männer sind gleichermaßen betroffen. Psoriasis ist eine chronische Hauterkrankung, die lebenslange Beschwerden verursacht. Etwa jeder fünfte Psoriasispatient leidet unter zusätzlichen arthritischen Beschwerden (Psoriasis arthritis) und weiteren chronischen entzündlichen Erkrankungen. Aufgrund des Zusammenspiels verschiedener Krankheitsbilder und - symptome kann die Lebenserwartung verkürzt sein. Für beide Hauterkrankungen sind Ursache und Auslöser noch nicht endgültig geklärt. In der Literatur werden unterschiedliche Theorien diskutiert. Es sollen genetische Faktoren, immunologische Veränderungen und/oder Umwelteinflüsse eine größere Rolle spielen.

Äußere Einflüsse können sehr vielfältig sein. Mechanische, infektiöse, medikamentöse, psychische und chronische Entzündungen gelten als Auslösefaktoren.

Für Psoriasis ist ein möglicher Erklärungsansatz der Erkrankung, dass aufgrund von Immunreaktionen das körpereigene Abwehrsystem gestört ist. Die Produktion der für die Körperabwehrreaktionen zuständigen T-Zellen verläuft dabei unkontrolliert, wobei die T-Zellenproduktion jedoch ein entscheidender Faktor in der Regulation des Abwehrsystems der Haut ist. Bei der Psoriasis bildet sich eine unkontrollierte Antikörperabwehr aus, die nicht nur körperfremde, sondern auch körpereigene Bestandteile bekämpft. Es kommt zur fehlgesteuerten Reproduktion der Hautzellen. Befallene Hautpartien können starke Reizungen, Rötungen, silbrig-schuppige Ablagerungen aufweisen und sind zum Teil verdickt. Einige Patienten weisen rissige Haut auf und haben offene Wundstellen.

Bei gesunder Haut erneuert sich die Oberhaut (Epidermis) in regelmäßigen Abständen. Dabei werden neue Hautzellen gebildet, die dann altern und verhornen. Die verhornten Hautzellen (Keratinozyten) werden vom Körper abgestoßen. Bei einem gesunden Körper verläuft dieser Vorgang nahezu unbemerkt und unsichtbar. Die Keratinozyten bilden bei der gesunden Haut ein natürliches Schutzschild gegenüber äußeren Umwelteinflüssen. Der Reparaturmechanismus gesunder Haut erfolgt durch gezielte Steuerung der Keratinozytenbildung und Aktivierung der T-Zellen. Bei der Psoriasis-Erkrankung ist das Zellwachstum hingegen gestört. Die Bildung der Hautzellen ist stark beschleunigt und überproportional viele Zellen werden gebildet. Die erhöhte Keratinozytenbildung wird ohne äußere Einwirkung aktiviert und ungesteuert fortgesetzt. Es bildet sich eine glänzende, silbrigweiße schuppige Schicht auf der Haut. Die unteren Hautschichten sind aufgrund des unkontrollierten Zellwachstums stark durchblutet und erscheinen dadurch stark gerötet.

Die krankhaften Hautveränderungen (Plaques) sind häufig einzeln inselförmig verteilt. Am häufigsten sind Hautpartien betroffen, die gedehnt werden und ständig mechanischem Stress ausgesetzt sind. Die Hautareale verdicken und schuppen sich. Durch die Schuppenbildung verhärtet sich die Haut und neigt zu Trockenheit und Wunden.

Eine Heilung der Psoriasis ist nicht möglich. Es gibt viele unterschiedliche Therapieansätze zur Linderung der Beschwerden. Die Therapien richten sich nach dem Beschwerdegrad, der Lokalisation und der Flächenausbreitung der Läsionen. Zur Anwendung kommen Lokaltherapien und systemische Therapien, die in Form von Präparaten oder physikalisch erfolgen können. Grundsätzlich werden bei anfänglichen Beschwerden und zur allgemeinen Pflege feuchtigkeitsspendende Hautpflegeprodukte in Form von Lotionen, Cremes, Ölen und Salben eingesetzt. Ist die Krankheit weiter fortgeschritten, werden äußerliche (topische), innerliche (systemische) Therapie und Licht als Therapieformen eingesetzt. All diesen Maßnahmen ist gemein, dass die Schuppenbildung und Entzündungsentwicklung unterdrückt werden soll. Ziel ist es, das normale Immunreaktionsgleichgewicht wieder herzustellen.

Jedoch ist es insbesondere bei einer systemischen Therapiebehandlung möglich, dass diese tiefgreifende Auswirkungen auf den Körper zeigt und eine äußerst gründliche Beobachtung und Kontrolle erfordert, da es zu schwerwiegenden Komplikationen und Nebenwirkungen kommen kann.

Es ist eine Aufgabe der vorliegenden Erfindung, eine Zusammensetzung zum Auftragen auf die Haut bereitzustellen, die die Nachteile aus dem Stand der Technik überwindet, insbesondere eine äußerst schonende Weise ermöglicht, die natürliche Schutzfunktion der Haut wieder herzustellen. Dabei soll insbesondere die natürliche Barrierefunktion der Haut regeneriert und die Abläufe gesunder Haut aufrechterhalten werden. Dabei soll die erfindungsgemäße Zusammensetzung bevorzugt zur Unterstützung der therapeutischen Behandlung von Psoriasis und Neurodermitis einsetzbar sein.

Diese Aufgabe wird gelöst durch eine Zusammensetzung zum Auftragen auf die Haut, die einen dermatologisch annehmbaren Träger und Kokosöl und Haselnussöl und/oder Avellanaöl, und Brennnesselöl umfasst.

Dabei ist bevorzugt vorgesehen, dass das Gewichtsverhältnis von dermatologisch annehmbarem Träger:Kokosöl:Haselnussöl und/oder Avellanaöl:Brennnesselöl in einem Bereich von 1-50:1-30:1-50:1-10 liegt.

Ferner ist bevorzugt vorgesehen, dass die Zusammensetzung Mandelöl umfasst.

Dabei ist bevorzugt vorgesehen, dass das Gewichtsverhältnis von dermatologisch annehmbarem Träger:Kokosöl:Haselnussöl und/oder Avellanaöl:Brennnesselöl:Mandelöl in einem Bereich von 1-50:1-30:1-50:1-10:1-20 liegt.

Ferner ist bevorzugt vorgesehen, dass die Zusammensetzung TRF-Extrakt (Tocotrienol-reiche Fraktion) umfasst.

Besonders bevorzugt ist dabei, dass das Gewichtsverhältnis von Träger:Kokosöl:Haselnussöl und/oder Avellanaöl: Brennnesselöl: Mandelöl: TRF-Extrakt in einem Bereich von 1-50:1-30:1-50:1-10:0-20:1-20 liegt.

Außerdem kann vorgesehen sein, dass die Zusammensetzung ferner Bittermandelöl umfasst.

Dabei ist bevorzugt vorgesehen, dass das Gewichtsverhältnis von Träger:Kokosöl:Haselnussöl und/oder Avellanaöl: Brennnesselöl: Mandelöl: TRF-Extrakt: Bittermandelöl in einem Bereich von 1-50:1-30:1-50:1-10:0-20:0-20:1-10 liegt.

Ferner wird vorgeschlagen, dass die Zusammensetzung natürliche Aromastoffe, vorzugsweise Lavendelaroma, umfasst.

Dabei ist bevorzugt vorgesehen, dass das Gewichtsverhältnis von Träger:Kokosöl:Haselnussöl und/oder Avellanaöl: Brennnesselöl: Mandelöl: TRF-Extrakt: Bittermandelöl:natürlicher Aromastoff in einem Bereich von 1-50:1-30:1-50:1-10:0-20:0-20:0-10:0,1-1 liegt.

Besonders bevorzugt ist vorgesehen, dass die Zusammensetzung umfasst:

| | | |
|---|---|---|
| Dermatologisch annehmbarer Träger | 1-50 Gew.-%, bevorzugt 30-50 Gew.-%, | |
| | | noch bevorzugter 40,00 Gew.-%, |
| Kokosöl | 1-30 Gew.-%, bevorzugt 10-30 Gew.-%, | |
| | | noch bevorzugter 15-25 Gew.-%, |
| | | noch bevorzugter 17,50 Gew.-%, |
| Haselnussöl und/oder Avellanaöl | 1-50 Gew.-%, bevorzugt 10-40 Gew.-%, | |
| | | noch bevorzugter 15-35 Gew.-%, |
| | | noch bevorzugter 20-30 Gew.-%, |
| | | noch bevorzugter 25,00 Gew.%, |
| Brennnesselöl | 1-10 Gew.-%, bevorzugt 1-7 Gew.-%, | |
| | | noch bevorzugter 3,00 Gew.-%, |
| Mandelöl | 0-20 Gew.%, bevorzugt 5-15 Gew.-%, | |
| | | noch bevorzugter 10,00 Gew.-%, |
| TRF-Extrakt (Tocotrienol-reiche Fraktion) | 0-20 Gew.-%, bevorzugt 1-10 Gew.-%, | |
| | | noch bevorzugter 2-7 Gew.-%, |
| | | noch bevorzugter 3,00 Gew.-%, |
| Bittermandelöl | 0-10 Gew.%, bevorzugt 0,5-3 Gew.-%, | |
| | | noch bevorzugter 1,00 Gew.-%, |
| Aromastoff | 0-1 Gew.-%, bevorzugt 0,3-0,7 Gew.-%, | |
| | | noch bevorzugter 0,50 Gew.-%, |

wobei sich alle Gewichtsprozentangaben auf die Gesamtmenge der Zusammensetzung beziehen.

Enthält die erfindungsgemäße Zusammensetzung dermatologisch annehmbare Träger, Kokosöl, Haselnussöl und/oder Avellanaöl, Brennnesselöl und Bittermandelöl, liegen die Gewichtsverhältnisse bevorzugt in einem Bereich von 1-50:1-30:1-50:1-10:1-10.

Weiterhin kann vorgesehen sein, dass sie in Form einer Salbe, Creme, Lotion, Tinktur, eines Öls oder eines Gels vorliegt.

Im Grunde genommen kann jeder für die Herstellung von Salben, Cremes, Lotionen, Tinkturen, Ölen oder Gelen geeigneter dermatologisch annehmbarer Träger verwendet werden. Fachleuten auf dem Gebiet sind entsprechende dermatologisch annehmbare Träger bekannt.

Es kann dabei bevorzugt vorgesehen sein, dass der dermatologisch annehmbare Träger ausgewählt ist aus den Gruppen:
a. der Hydrophoben Salben
   z.B. bestehend aus: Weiße Vaseline Ph. Eur., Gelbe Vaseline Ph. Eur, Einfache Augensalbe DAC
b. der Lipophilen Gelen
   z.B. bestehend aus: Hydrophobes Basisgel DAC
c. der Lipogelen
   z.B. bestehend aus: Schweineschmalz DAB, Weiße Mandelölsalbe FH A.4, Excipial Mandelöl-Salbe
d. der Wasseraufnehmenden Salben W/O Absorptionssalben
   z.B. bestehend aus: Wollwachsalkohol-Salbe DAB (Ungt. Alcohol. Lanae), Eucerinum abhydricum, Ungt. Sorbitansesquioleati, Ungt. Sorbitanmonostearinic, Wollwachsfreie W/O-Absorptionssalbe, Pionier KWH pharma, Emulgierendes hydrophobes Basisgel DAC, Emulgierende Augensalbe (NRF 15.20)
e. der O/W Absorptionssalben
   z.B. bestehend aus: Hydrophile Salbe DAB, Unguentum Cordes
f. der Lipophilen Cremes
   z.B. bestehend aus: Lanolin DAB, Wasserhaltige Wollwachsalkoholsalbe (Ungt. Alcohol. Lanae aquos.), Eucerin cum aqua, Weiche Salbe (Ungt. Molle) DAC, Hydrophobe Basiscreme DAC (NRF 11.104), Hydrophobe Tretinoin-Creme 0,025/0,05 oder 0,1 % (NRF 11.123), Hydrophobe Triclosan-Creme 2% (NRF 11.122), Hydrophobe Polidocanol-Creme 5% (NRF 11.119), Hydrophobe Polidocanol-Creme 5% mit Harnstoff 5% (NRF 11.120), Cremor vaselini MB 59, Cremor sorbitansequioleati, Cremor sorbitanmonostearati,
g. der W/O-Lotionen
h. der Quasi-W/O-Cremes
   z.B. bestehend aus: Kühlsalbe (Ungt. Leniens) DAB, Cold cream naturel RP
i. der Hydrophilen Cremes
   z.B. bestehend aus: Nichtionische hydrophile Creme DAB, Nichtionische hydrophile Creme SR DAC (NRF S.27), Nichtionisches wasserhaltiges Liniment DAC (NRF 11.92)
j. der Hydrophilen Lotionen
   z.B. bestehend aus: Hydrophile Basisemulsion (NRF S.25)
k. der Hydrophilen Gele
   z.B. bestehend aus: Hydroxyethylcellulose - Gel DAB

Eine zweite Aufgabe wird durch eine Verwendung der Zusammensetzung zur Behandlung von Hauterkrankungen gelöst, insbesondere Psoriasis, Neurodermitis (atopische Dermatitis), seborrhoeische Dermatitis, Urticaria, Erythem und Lichen planus sowie zur Behandlung von Wunden / Hautverbrennungen sowie Hühneraugen.

Überraschenderweise wurde gefunden, dass die erfindungsgemäße Zusammensetzung mit Hauterkrankungen, wie insbesondere Psoriasis und Neurodermitis, verbundene Beschwerden lindert. Außerdem beschleunigt die erfindungsgemäße Zusammensetzung die Verheilung von Wunden / Hautverbrennungen sowie Hühneraugen. Dies erfolgt nach Ansicht der Erfinder aufgrund physikalischer Effekte. Die Zusammensetzung basiert auf natürlichen Ölen sowie einem üblichen Trägergrundstoff zur Herstellung der Zusammensetzung, um diese für eine topische Auftragung geeignet zu machen. Die Inhaltsstoffe wirken sich in Kombination positiv auf die Regeneration der natürlichen Hautfunktionen aus. Die Linderung erfolgt vielmehr durch feuchtigkeitsspendende und pflegende Effekte. Die Zusammensetzung erzeugt beim Auftragen auf die Haut einen Schutzfilm, der die befallenen Hautpartien vor äußeren Umwelteinflüssen schützt und die körpereigene Regeneration der Hautfunktionen unterstützt. Durch Bildung eines Schutzfilms wird die erhöhte Austrocknung der Läsionen gestoppt, und es kann sich der Wassergehalt in den Hautschichten regenerieren. Insbesondere der Wassergehalt in der Hornschicht (Stratum corneum) ist dabei ein entscheidender Faktor für eine gesunde Haut. Die Epidermis der gesunden Haut hat natürliche Barrierefunktionen, welche den Wasserhaushalt regulieren und die Haut vor Umwelteinflüssen sowie hautschädlichen Substanzen schützen. Ist die Haut jedoch von Psoriasis oder Neurodermitis befallen, ist die natürliche Barrierefunktion gestört. Die erfindungsgemäße Zusammensetzung beschleunigt die Regeneration der normalen Barrierefunktion der Haut. Die erfindungsgemäße Zusammensetzung schützt die Haut vor schädlichen Umwelteinflüssen und Allergie auslösenden Substanzen. Die in der erfindungsgemäßen Zusammensetzung enthaltenen Lipidkomponenten erzeugen zudem einen kühlenden Effekt, der eine zusätzliche Linderung erzeugt.

Die Effekte der erfindungsgemäßen Zusammensetzung bewirken, dass sich die Haut regenerieren kann, die Bildung der natürlichen Barrierefunktion der Haut unterstützt wird und die natürliche Barriereschutzfunktion gesunder Haut wieder hergestellt wird.

Weitere Merkmale und Vorteile der erfindungsgemäßen Zusammensetzung ergeben sich aus der folgenden detaillierten Beschreibung von bevorzugten Ausführungsformen.

Beispielherstellung einer Zusammensetzung als Creme:
In einen dermatologisch annehmbaren Träger, hier im Beispiel Eucerin anhydricum, werden die Komponenten, die in der Zusammensetzung aufgeführten Öle, nacheinander unter rühren beigemischt und in den Träger eingearbeitet. Die Mengenverhältnisse der Komponenten ergeben sich aus der Anzahl der zu verwendenden Bestandteile und der Ansatzgröße. Die Mengenanteile ergeben sich aus der gewünschten Ansatzgröße. Die Gewichtsmengen errechnen sich aus den Gewichtsprozenten im Verhältnis zur Ansatzgröße.
Je nach verwendeten dermatologischem Träger und Anzahl bzw. Mengenverhältnis der eingesetzten Öle ergibt sich eine ölige oder cremige Struktur der Zusammensetzung.

Verwendete Zusammensetzungen in Gewichtsprozent zur Testung der Wirksamkeit:

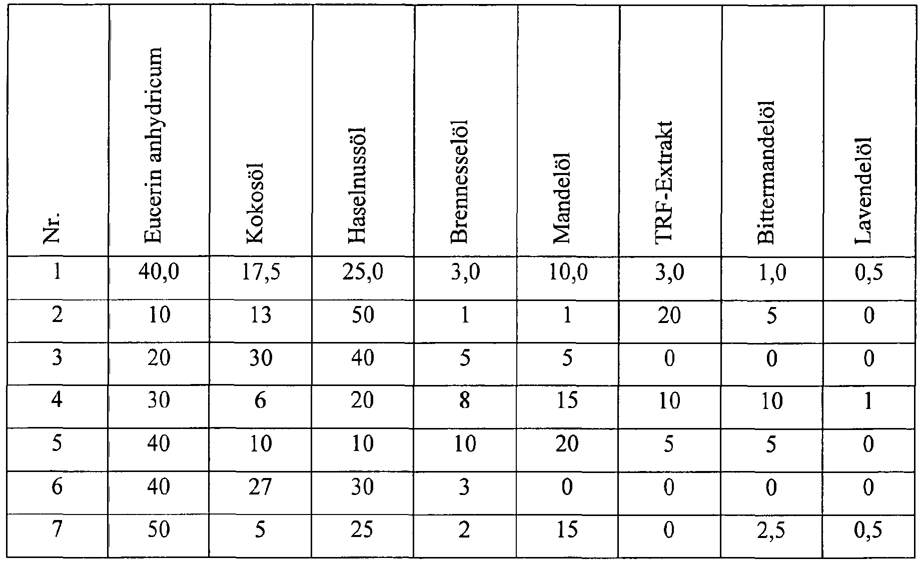

Die in der Tabelle aufgeführten Creme-Zusammensetzungen wurden zum Wirksamkeitstest an 49 Patienten mit Psoriasis, 33 Patienten mit Neurodermitis sowie 28 Patienten zur Behandlung von Wunden / Hautverbrennungen sowie Hühneraugen angewendet. Die Creme mit den Beispielzusammensetzungen (s. oben) wurden 1 - 3 mal täglich aufgetragen.

Die Wirkung der Zusammensetzungen auf die erkrankte Haut wurde bei 49 Patienten mit Psoriasis in Abständen von 1, 5, 10, 20, 30, 45, 60, 75 und 90 Tagen nach Beginn der Auftragung beurteilt.

In der folgenden Tabelle sind die Ergebnisse der beobachteten Wirkung aufgelistet.

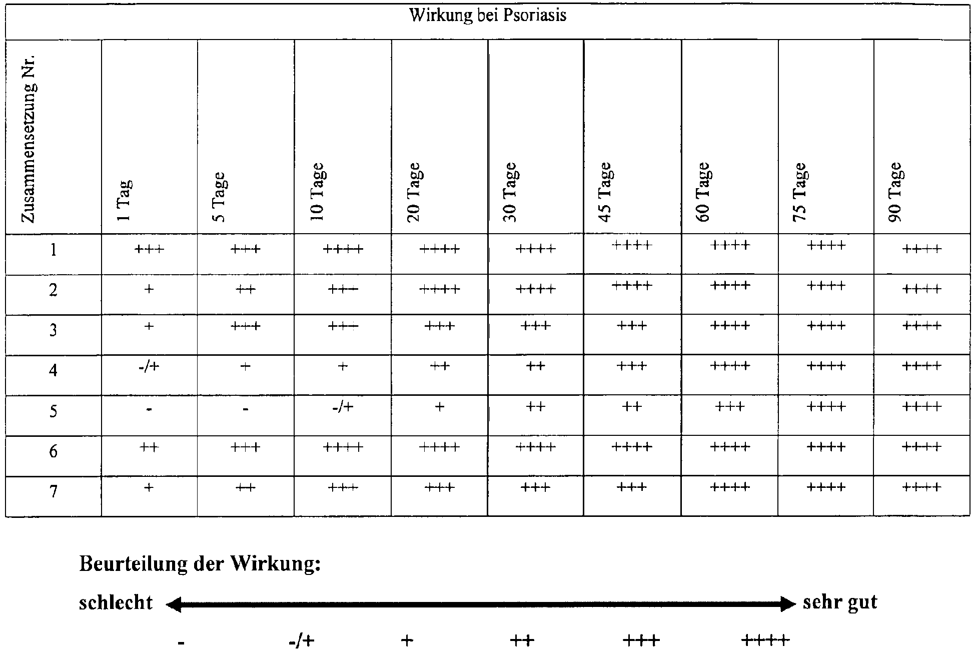

Die Wirkung der Zusammensetzungen auf die erkrankte Haut wurde bei 33 Patienten mit Neurodermitis in Abständen von 5, 10, 14, 22, 30, 45, 60, 75 und 90 Tagen nach Beginn der Auftragung beurteilt.

In der folgenden Tabelle sind die Ergebnisse der beobachteten Wirkung aufgelistet.

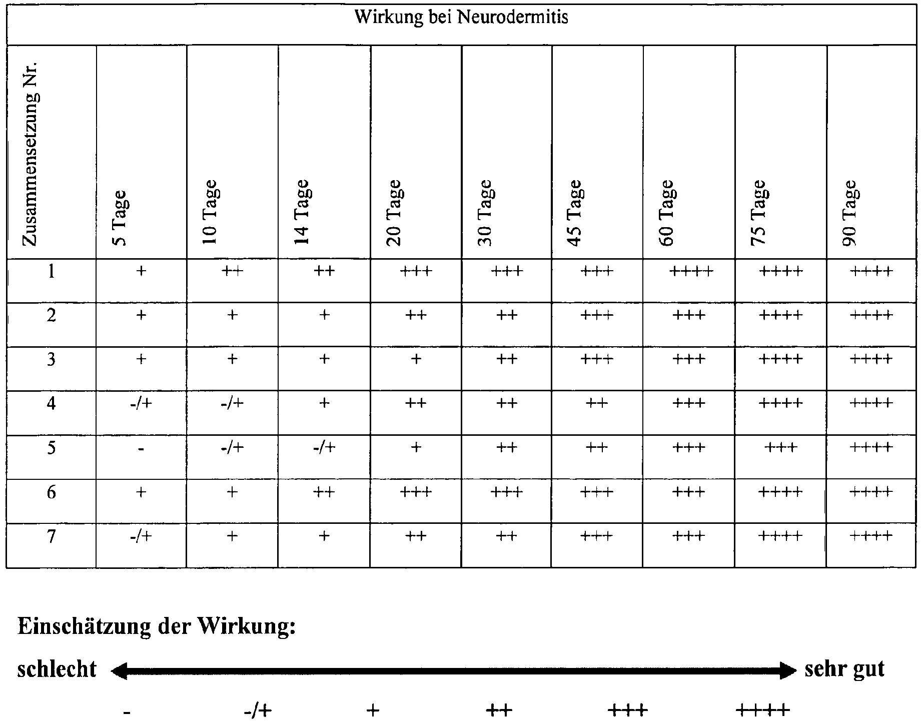

Die Wirkung der Zusammensetzungen auf die erkrankte Haut wurde bei 28 Patienten mit Wunden / Hautverbrennungen sowie Hühneraugen in Abständen von 1, 2, 5, 7, 9, 10 und 14 Tagen nach Beginn der Auftragung beurteilt.

In der folgenden Tabelle sind die Ergebnisse der beobachteten Wirkung aufgelistet.

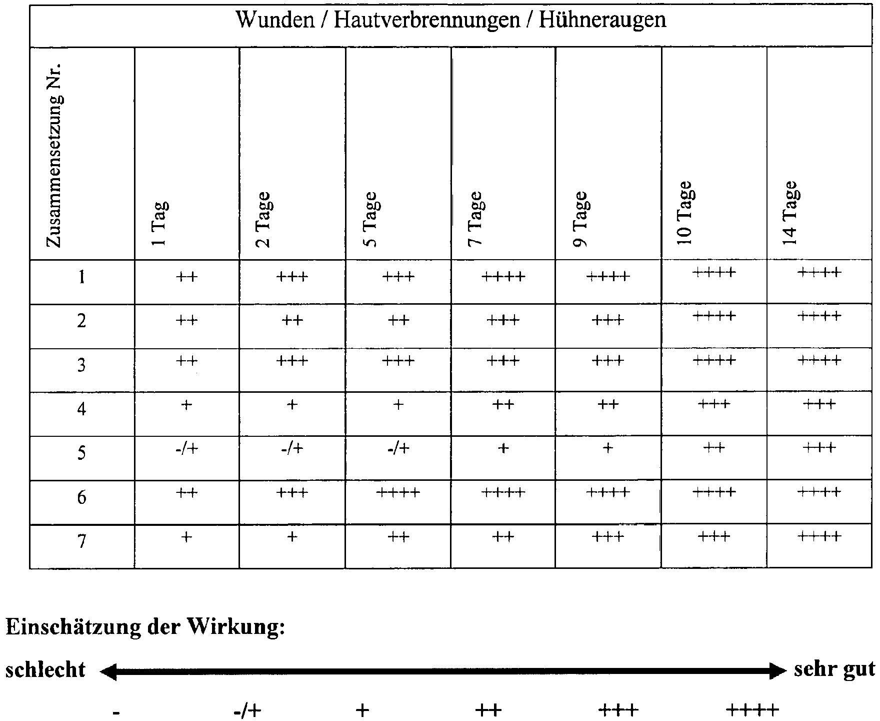

Aus den oben aufgeführten Ergebnissen der Zusammensetzungen lässt sich entnehmen, dass alle aufgeführten Zusammensetzungen eine Linderung bei Psoriasis, Neurodermitis und bei der Behandlung von Wunden / Hautverbrennungen sowie Hühneraugen bewirken. Die bevorzugt vorgesehene Zusammensetzung hat die höchste universellste und übergreifendste Wirksamkeit bei allen vorgesehenen Anwendungsgebieten.

Die in der vorstehenden Beschreibung und in den Ansprüchen offenbarten Merkmale der Erfindung können sowohl einzeln als auch in jeder beliebigen Kombination für die Verwirklichung der Erfindung in ihren einzelnen Ausführungsformen wesentlich sein.

## Patentansprüche

1. Zusammensetzung zum Auftragen auf die Haut, die einen dermatologisch annehmbaren Träger und Kokosöl und Haselnussöl und/oder Avellanaöl, und Brennnesselöl umfasst.

2. Zusammensetzung nach Anspruch 1, **dadurch gekennzeichnet, dass** sie ferner TRF-Extrakt (Tocotrienol-reiche Fraktion) umfasst.

3. Zusammensetzung nach Anspruch 1 oder 2, **dadurch gekennzeichnet, dass** das Gewichtsverhältnis von dermatologisch annehmbarem Träger:Kokosöl:Haselnussöl und/oder Avellanaöl:Brennnesselöl in einem Bereich von 1-50:1-30:1-50:1-10 liegt.

4. Zusammensetzung nach einem der vorangehenden Ansprüche, **dadurch gekennzeichnet, dass** sie ferner Mandelöl umfasst.

5. Zusammensetzung nach Anspruch 4, **dadurch gekennzeichnet, dass** das Gewichtsverhältnis von Träger:Kokosöl:Haselnussöl und/oder Avellanaöl: Brennnesselöl:Mandelöl in einem Bereich von 1-50:1-30:1-50:1-10:1-20 liegt.

6. Zusammensetzung nach einem der vorangehenden Ansprüche, **dadurch gekennzeichnet, dass** sie ferner Bittermandelöl umfasst.

7. Zusammensetzung nach Anspruch 6, **dadurch gekennzeichnet, dass** das Gewichtsverhältnis von Träger:Kokosöl:Haselnussöl und/oder Avellanaöl:Brennnesselöl:Mandelöl:Bittermandelöl in einem Bereich von 1-50:1-30:1-50:1-10:0-20:1-10 liegt.

8. Zusammensetzung nach einem der vorangehenden Ansprüche, **dadurch gekennzeichnet, dass** sie ferner natürliche Aromastoffe, vorzugsweise Lavendelaroma, umfasst.

9. Zusammensetzung nach einem der vorangehenden Ansprüche, welche umfasst:
| | | |
|---|---|---|
| Dermatologisch annehmbarer Träger | 1-50 Gew.-%, bevorzugt 30-50 Gew.-%, | |
| | | noch bevorzugter 40,00 Gew.-%, |
| Kokosöl | 1-30 Gew.-%, bevorzugt 10-30 Gew.-%, | |
| | | noch bevorzugter 15-25 Gew.-%, |
| | | noch bevorzugter 17,50 Gew.-%, |
| Haselnussöl und/oder Avellanaöl | 1-50 Gew.-%, bevorzugt 10-40 Gew.-%, | |
| | | noch bevorzugter 15-35 Gew.-%, |
| | | noch bevorzugter 20-30 Gew.-%, |
| | | noch bevorzugter 25,00 Gew.%, |
| Brennnesselöl | 1-10 Gew.-%, bevorzugt 1-7 Gew.-%, | |
| | | noch bevorzugter 3,00 Gew.-%, |
| Mandelöl | 0-20 Gew.-%, bevorzugt 5-15 Gew.-%, | |
| | | noch bevorzugter 10,00 Gew.-%, |
| TRF-Extrakt (Tocotrienol-reiche Fraktion) | 0-20 Gew.-%, bevorzugt 1-10 Gew.-%, | |
| | | noch bevorzugter 2-7 Gew.-%, |
| | | noch bevorzugter 3,00 Gew.-%, |
| Bittermandelöl | 0-10 Gew.-%, bevorzugt 0,5-3 Gew.-%, | |
| | | noch bevorzugter 1,00 Gew.-%, |
| Aromastoff | 0-1 Gew.-%, bevorzugt 0,3-0,7 Gew.-%, | |
| | | noch bevorzugter 0,50 Gew.-%, |
wobei sich alle Gewichtsprozentangaben auf die Gesamtmenge der Zusammensetzung beziehen.

10. Zusammensetzung nach einem der vorangehenden Ansprüche, **dadurch gekennzeichnet, dass** sie in Form einer Salbe, Creme, Lotion, Tinktur, eines Öls oder eines Gels vorliegt.

11. Zusammensetzung nach einem der vorangehenden Ansprüche, **dadurch gekennzeichnet, dass** der dermatologisch annehmbare Träger ausgewählt ist aus den Gruppen:
a. der Hydrophoben Salben
b. der Lipophilen Gelen
c. der Lipogelen
d. der Wasseraufnehmenden Salben W/O Absorptionssalben
e. der O/W Absorptionssalben
f. der Lipophilen Cremes
g. der W/O-Lotionen
h. der Quasi-W/O-Cremes
i. der Hydrophilen Cremes
j. der Hydrophilen Lotionen
k. der Hydrophilen Gele

12. Zusammensetzung nach einem der Ansprüche 1 bis 10 zur Verwendung in der Behandlung von Hauterkrankungen, insbesondere Psoriasis, Neurodermitis, atopische Dermatitis, seborrhoeische Dermatitis, Urticaria, Erythem und Lichen planus sowie zur Behandlung von Wunden / Hautverbrennungen sowie Hühneraugen.

## Claims

1. Compound for skin application comprising a dermatologically acceptable carrier and coconut oil and hazelnut oil and/or avellana oil, and stinging nettle oil.

2. Compound according to claim 1, **characterised in that** it also comprises TRF (tocotrienol-rich fraction) extract.

3. Compound according to claim 1 or 2, **characterised in that** the weight ratio of dermatologically acceptable carrier: coconut oil: hazelnut oil and/or avellana oil: stinging nettle oil lies in a range of 1-50:1-30:1-50:1-10.

4. Compound according to one of the preceding claims, **characterised in that** it also comprises almond oil.

5. Compound according to claim 4, **characterised in that** the weight ratio of carrier:coconut oil:hazelnut oil and/or avellana oil:stinging nettle oil:almond oil lies in a range of 1-50:1-30:1-50:1-10:1-20.

6. Compound according to one of the preceding claims, **characterised in that** it also comprises bitter almond oil.

7. Compound according to claim 6, **characterised in that** the weight ratio of carrier: coconut oil: hazelnut oil and/or avellana oil: stinging nettle oil: almond oil: bitter almond oil lies in a range of 1-50:1-30:1-50:1-10:0-20:1-10.

8. Compound according to one of the preceding claims, **characterised in that** it also comprises natural aromatic substances, preferably lavender aroma.

9. Compound according to one of the preceding claims, which comprises:
| | | |
|---|---|---|
| Dermatologically acceptable carrier | 1-50% by weight, preferably 30-50% by weight, | |
| | | more preferably 40.00% by weight, |
| coconut oil | 1-30% by weight, preferably 10-30% by weight, | |
| | | more preferably 15-25% by weight, |
| | | more preferably 17.50% by weight, |
| hazelnut oil and/or avellana oil | 1-50% by weight, preferably 10-40% by weight, | |
| | | more preferably 15-35% by weight, more preferably 20-30% by weight, |
| | | more preferably 25.00% by weight, |
| stinging nettle oil | 1-10% by weight, preferably 1-7% by weight, | |
| | | more preferably 3.00 % by weight, |
| almond oil | 0-20% by weight, preferably 5-15% by weight, | |
| | | more preferably 10.00 % by weight, |
| TRF (tocotrienol-rich fraction) extract | 0-20% by weight, preferably 1-10% by weight, | |
| | | more preferably 2-7% by weight, |
| | | more preferably 3.00% by weight, |
| bitter almond oil | 0-10% by weight, preferably 0.5-3% by weight, | |
| | | more preferably 1.00% by weight, |
| aromatic substance | 0-1% by weight, preferably 0.3-0.7% by weight, | |
| | | more preferably 0.50% by weight, |
wherein all percentages by weight relate to the total quantity of the compound.

10. Compound according to one of the preceding claims, **characterised in that** it exists in the form of an ointment, a cream, a lotion, a tincture, an oil or a gel.

11. Compound according to one of the preceding claims, **characterised in that** the dermatologically acceptable carrier is selected from the following groups:
a. hydrophobic ointments
b. lipophilic gels
c. lipogels
d. hydrophilic ointments W/O absorption ointments
e. O/W absorption ointments
f. lipophilic creams
g. W/O lotions
h. quasi W/O creams
i. hydrophilic creams
j. hydrophilic lotions
k. hydrophilic gels.

12. Compound according to one of the claims 1 to 10 for use in the treatment of skin diseases, especially psoriasis, neurodermatitis, atopical dermatitis, seborrhoeic dermatitis, urticaria, erythema and lichen planus and for the treatment of wounds/skin burns and corns.

## Revendications

1. Composition pour application sur la peau, comprenant un véhicule dermatologiquement acceptable et de l'huile de noix de coco et de l'huile de noisette et/ou de l'huile de *Corylus avellana* et de l'huile d'ortie.

2. Composition selon la revendication 1, **caractérisée en ce qu'**elle comprend en outre l'extrait TRF (fraction riche en tocotriénols).

3. Composition selon la revendication 1 ou 2, **caractérisée en ce que** le rapport en poids véhicule dermatologiquement acceptable : huile de noix de coco : huile de noisette et/ou huile de *Corylus avellana* : huile d'ortie est dans une plage de 1-50:1-30:1-50:1-10.

4. Composition selon l'une quelconque des revendications précédentes, **caractérisée en ce qu'**elle comprend en outre de l'huile d'amande.

5. Composition selon la revendication 4, **caractérisée en ce que** le rapport en poids véhicule : huile de coco: huile de noisette et/ou huile de *Corylus avellana* : huile d'ortie: huile d'amande est dans une plage de 1-50:1-30:1-50:1-10:1-20.

6. Composition selon l'une quelconque des revendications précédentes, **caractérisée en ce qu'**elle comprend en outre l'huile d'amande amère.

7. Composition selon la revendication 6, **caractérisée en ce que** le rapport en poids véhicule : huile de coco : huile de noisette et/ou huile de *Corylus avellana* : huile d'ortie : huile d'amande : huile d'amande amère est dans une plage de 1-50:1-30 :1-50 :1-10 :0-20 :1-10.

8. Composition selon l'une quelconque des revendications précédentes, **caractérisée en ce qu'**elle comprend en outre des arômes naturels, comprenant de préférence l'arôme de lavande.

9. Composition selon l'une quelconque des revendications précédentes, comprenant:
| | |
|---|---|
| le véhicule dermatologiquement acceptable | 1-50 % en poids, de préférence 30-50 % en poids, |
| | de préférence 40,00 % en poids, |
| huile de coco | 1-30 % en poids, de préférence 10-30 % en poids, |
| | plus préférentiellement encore de 15 à 25 % en poids, |
| | encore plus préférentiellement 17,50 % en poids, |
| huile de noisette et/ou huile de *Corylus avellana* | 1-50 % en poids, de préférence 10-40 % en poids, |
| | plus préférentiellement encore de 15 à 35 % en poids, |
| | plus préférentiellement encore de 20 à 30 % en poids, |
| | plus préférentiellement encore 25,00 % en poids, |
| huile d'ortie | 1-10 % en poids, de préférence 1-7 % en poids, |
| | plus préférentiellement 3,00 % en poids, |
| huile d'amande | 0-20 % en poids, de préférence 5-15 % en poids, |
| | encore plus préférentiellement 10,00 % en poids, |
| extrait TRF (fraction riche en tocotriénol) | 0-20 % en poids, de préférence 1-10 % en poids, |
| | plus préférablement 2-7 % en poids, |
| | encore plus préférentiellement 3,00 % en poids, |
| huile d'amande amère | 0-10 % en poids, de préférence 0,5-3 % en poids, |
| | plus préférablement 1,00 % en poids |
| arôme | 0-1 % en poids, de préférence 0,3-0,7 % en poids, |
| | plus préférablement, 0,50% en poids, |
dans laquelle tous les pourcentages en poids sont basés sur la quantité totale de la composition.

10. Composition selon l'une quelconque des revendications précédentes, **caractérisée en ce qu'**elle se présente sous la forme d'une pommade, crème, lotion, de teinture, de l'huile ou d'un gel.

11. Composition selon l'une quelconque des revendications précédentes, **caractérisée en ce que** le véhicule dermatologiquement acceptable est choisi parmi les groupes :
a. des pommades hydrophobes
b. des gels lipophiles
c. des lipogels
d. d'onguents récepteurs d'eau parmi les onguents d'absorption du type eau dans l'huile
e. d'onguents d'absorption du type huile dans l'eau
f. des crèmes lipophiles
g. des lotions du type eau dans l'huile
h. des crèmes quasi du type eau dans l'huile
i. des crèmes hydrophiles
j. des lotions hydrophiles
k. des gels hydrophiles

12. Utilisation de la composition selon l'une quelconque des revendications 1 à 10 pour le traitement de maladies de la peau, en particulier le psoriasis, l'eczéma, la dermatite atopique, la dermatite séborrhéique, l'urticaire, l'érythème et le lichen plan, ainsi que pour le traitement des plaies/brûlures et les cors.
